# FASCICULE DE BREVET EUROPEEN

(11) **EP 3 525 702 B1**
(45) Date de publication et mention de la délivrance du brevet: **15.12.2021**
(21) Numéro de dépôt: 17797385.6
(22) Date de dépôt: 12.10.2017
(51) Int. Cl.: A61B 17/88

(54) **IMPLANT MEDICAL MODULAIRE PERMETTANT UNE INJECTION CIBLEE**
MODULARES MEDIZINISCHES IMPLANTAT MIT EINER GEZIELTEN INJEKTION
MODULAR MEDICAL IMPLANT ALLOWING A TARGETED INJECTION

(30) Priorité: 13.10.2016 FR 1601491
(43) Date de publication de la demande: 21.08.2019
(73) Titulaire: Innoprod Medical, 31830 Plaisance-du-Touch (FR)
(72) Inventeur: LEGAY, Philippe Alain Lucien Fernand, 76530 Yville sur Seine (FR); PEYRE, Frédéric, 31530 Lasserre (FR)
(74) Mandataire: Icosa
(86) Numéro de dépôt international: PCT/FR2017/000191
(87) Numéro de publication internationale: WO 2018/069583

(56) Documents cités:
- WO-A1-2015/123733

## Description

La présente invention concerne un implant médical modulaire permettant l'injection ciblée d'un matériau liquide ou pâteux dans une cavité osseuse, favorisant ainsi le comblement et/ou la consolidation, sans risque de contraindre tout nerf, et/ou toute autre partie molle, pouvant être présent à proximité du site d'injection.

L'art antérieur propose divers matériels employés en ostéosynthèse, clous et vis, canulés de façon à être guidés lors de la pose, comme exposé dans WO2015/123733 A1.

Ces différents matériels peuvent être utilisés pour conduire un ciment acrylique, par l'intermédiaire de leur lumière, mais en aucun cas ne permettent de cibler le ou les sites d'injection.

Les matériels utilisés actuellement par les praticiens n'étant pas dédiés à cette technique,
soit ne permettent pas d'injecter, obligeant ainsi à effectuer des injections différenciées, en pratiquant d'autres voies d'abord, donc des actions supplémentaires préjudiciables pour le patient,
soit ne permettent pas d'éviter les nerfs ou les parties molles passant dans la cavité, à proximité de l'implant, donnant un risque de douleurs au patient.

La présente invention vise à donner la possibilité à l'intervenant de résoudre ces problèmes, par l'utilisation d'un implant médical modulaire qui permet, grâce à la combinaison de divers modules, de répondre aux besoins de la pratique, et notamment de cibler les zones, déterminées préalablement, devant recevoir le produit de comblement et/ou de consolidation.

Ce but est atteint grâce à un ensemble de modules, pouvant être solidarisés les uns aux autres dans un ordre prédéterminé, de façon à placer certains desdits modules dotés de perforations latérales à des distances requises, permettant de laisser échapper le produit d'injection aux emplacements ciblés.
Avantageusement, l'implant ainsi composé peut former un clou, une broche ou une vis de longueur nécessaire, caractérisé
- en ce que chacun des modules le constituant est de forme tubulaire dont la section peut être circulaire, dotée ou non de faces planes sur tout ou partie de sa longueur, carrée ou polygonale.
Favorablement, les faces planes seraient appelées à coopérer avec des outils de serrage,
- en ce que lesdits modules sont répartis en modules distaux, modules intermédiaires et modules proximaux,
- en ce que certains des modules intermédiaires sont dotés de perforations traversant de la lumière interne jusqu'à la périphérie, pouvant être orientées ascendantes, descendantes ou perpendiculaires à ladite lumière interne,
- en ce que lesdits modules peuvent être de longueurs différentes, de façon à optimiser toutes les possibilités de longueur de l'implant final et le choix du positionnement du ou des modules perforés,
- en ce que chaque module distal peut se terminer par un filetage à os auto-perforant et auto-taraudant et comporte un principe de solidarisation femelle appelé à coopérer avec le principe de solidarisation mâle de l'un ou l'autre des modules intermédiaires ou proximaux,
- en ce que chaque module proximal débute par un filetage à os auto-perforant et auto-taraudant et se termine par un principe de solidarisation mâle, appelé à coopérer avec le principe de solidarisation femelle de l'un ou l'autre des modules intermédiaires ou distaux,
- en ce que chacun des modules intermédiaires débute par un principe de solidarisation femelle et se termine par un principe de solidarisation mâle,
Suivant le mode de réalisation souhaité, chaque principe de solidarisation mâle peut être un filetage mécanique ou un (ou plusieurs) ergot(s) périphérique(s), ou tout autre moyen mécanique.
Réciproquement, chaque principe de solidarisation femelle peut être un taraudage mécanique ou une (ou plusieurs) gorge(s) périphérique(s), ou tout autre moyen mécanique.
Avantageusement, chaque principe de solidarisation mâle de l'un ou l'autre des différents modules est appelé à coopérer indifféremment avec le principe de solidarisation femelle de l'un ou l'autre des autres modules, de façon à positionner les modules perforés aux niveaux adéquat et obtenir la longueur désirée de l'implant médical modulaire.

L'invention sera bien comprise et d'autres caractéristiques et avantages de celle-ci apparaîtront en référence aux dessins schématiques annexés représentant, à titre d'exemple non limitatif, une forme de réalisation de l'implant pour injection ciblée qu'elle concerne.
- la figure 1 représente les quatre types de modules nécessaires à la réalisation d'un implant médical modulaire,
- la figure 2 représente une des réalisations possibles d'un implant médical modulaire,
- la figure 3 représente une coupe d'un module intermédiaire perforé.

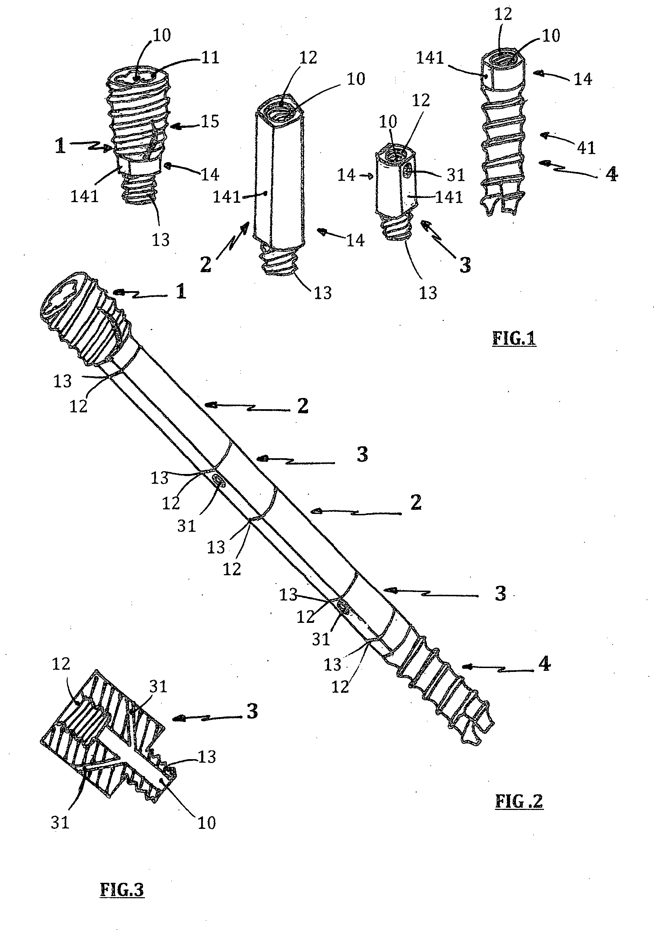

La figure 1 représente les quatre types de modules nécessaires à la réalisation d'un implant médical modulaire, dont :
Le module proximal 1 se compose d'un principe de solidarisation mâle 13 appelé à coopérer avec le principe de solidarisation femelle 12 d'un module intermédiaire 2 ou d'un module intermédiaire perforé 3 ou d'un module distal 4, surmonté d'un corps 14 pouvant être doté de faces planes 141 appelées à recevoir un outil de serrage, puis d'un filetage à os 15 à l'intérieur duquel est usiné une empreinte femelle 11 appelée à être actionnée par un tournevis adéquat.

Les modules intermédiaires 2 se composent d'un principe de solidarisation mâle 13 appelé à coopérer avec un principe de solidarisation femelle 12 d'un autre module intermédiaire 2 ou d'un module intermédiaire perforé 3 ou d'un module distal 4, surmonté d'un corps 14 pouvant être doté de faces planes 141 appelées à recevoir un outil de serrage, et d'un principe de solidarisation femelle 12 appelé à coopérer avec un principe de mâle 13 d'un module proximal 1 ou d'un module intermédiaire 2 ou d'un module intermédiaire perforé 3.
Avantageusement lesdits modules intermédiaires 2 peuvent être de longueur différentes de façon à réaliser la longueur nécessaire de l'implant médical modulaire, tout en plaçant le ou les modules intermédiaires perforés 3 aux niveaux adéquats pour permettre de laisser échapper le produit d'injection aux emplacements ciblés.

Les modules intermédiaires perforés 3 sont composés suivant le même principe que les modules intermédiaires 2 et sont dotés de perforations 31 traversant de la lumière interne 10 jusqu'à la périphérie du corps 14.
Utilement, lesdites perforations 31 peuvent être orientées perpendiculairement à la lumière interne 10, ascendantes débouchant vers le principe de solidarisation femelle 12 ou descendante débouchant vers le principe de solidarisation mâle 13,

Le module distal 4 se compose d'un filetage à os 41 surmonté d'un corps 14 pouvant être doté de faces planes 141 appelées à recevoir un outil de serrage, et d'un principe de solidarisation femelle 12 appelé à coopérer avec un principe de solidarisation mâle 13 d'un module intermédiaire 2 ou d'un module intermédiaire perforé 3 ou d'un module proximal 1.
Utilement, tous les modules sont traversés longitudinalement de lumières 10 de même diamètre assurant une continuité depuis le module proximal jusqu'au module distal, lors de la composition de l'implant modulaire, permettant la conduction d'un matériau liquide ou pâteux depuis l'empreinte femelle 11 jusqu'aux perforations 31.

La figure 2 représente une des réalisations possibles d'un implant médical modulaire composé, à titre d'exemple, d'un module proximal 1 dont le principe de solidarisation mâle 13 coopère avec le principe de solidarisation femelle 12 d'un module intermédiaire 2 dont le principe de solidarisation mâle 13 coopère avec le principe de solidarisation femelle 12 d'un module intermédiaire perforé 3 dont le principe de solidarisation mâle 13 coopère avec le principe de solidarisation femelle 12 d'un module intermédiaire 2 dont le principe de solidarisation mâle 13 coopère avec le principe de solidarisation femelle 12 d'un module intermédiaire perforé 3 dont le principe de solidarisation mâle 13 coopère avec le principe de solidarisation femelle 12 d'un module distal 4.
Avantageusement, les modules intermédiaires 2 peuvent être de longueurs différentes de façon à réaliser la longueur nécessaire de l'implant médical modulaire, tout en plaçant le ou les modules intermédiaires perforés 3 aux niveaux adéquats pour permettre de laisser échapper le produit d'injection aux emplacements ciblés.

La figure 3 représente une vue en coupe d'un module intermédiaire perforé 3 dont les perforations 31 peuvent être orientées perpendiculairement à la lumière interne 10 ou obliques, ascendantes depuis la lumière 10 vers le principe de solidarisation femelle 12 ou descendante depuis la lumière 10 vers le principe de solidarisation mâle 13.

Comme cela apparaît de tout ce qui précède, l'invention fournit un implant médical modulaire, notamment d'ostéosynthèse, permettant une injection ciblée et présentant, par rapport aux matériels homologues de la technique antérieure ou empruntés à d'autres disciplines, l'avantage déterminant de permettre de cibler le ou les sites d'injection d'un matériau liquide ou pâteux dans une cavité osseuse, en particulier pour réaliser un comblement et/ou une consolidation dans les meilleures conditions, en évitant tout risque de contraindre tout nerf et/ou toute autre partie molle pouvant être présent à proximité du site d'injection.

Il va de soi que l'invention n'est pas limitée à la forme de réalisation décrite ici à titre d'exemple, mais qu'elle s'étend à tous les matériels et toutes les formes de réalisations couvertes par les revendications ci-après annexées.

## Revendications

1. Implant médical modulaire permettant une injection ciblée composé d'un module proximal (1), d'un ou plusieurs modules intermédiaires (2) dont un ou plusieurs sont des modules intermédiaires perforés (3) munis de perforations (31), et d'un module distal (4), **caractérisé en ce que** chacun des modules (1, 2, 3, 4) a une lumière (10) et tous les modules nécessaires à la réalisation de la longueur souhaitée de l'implant médical modulaire sont solidarisés les uns aux autres par l'intermédiaire d'ensembles coopérants comprenant un principe de solidarisation mâle (13) et un principe de solidarisation femelle (12), permettant ainsi de positionner les perforations (31) du ou des modules intermédiaires perforés (3) aux niveaux requis pour cibler l'injection d'un matériau liquide ou pâteux.

2. Implant médical modulaire permettant une injection ciblée suivant la revendication **1, caractérisé en ce qu'**il comprend des modules (1, 2, 3, 4) de longueurs différentes, de façon à optimiser toutes les possibilités de longueur de l'implant final et le choix du positionnement du ou des modules perforés (3).

3. Implant médical modulaire permettant une injection ciblée suivant l'une quelconque des revendications **1** ou **2, caractérisé en ce que** chacun des modules (1, 2, 3, 4) le constituant est un tube définissant la lumière (10) et ayant un corps (14) dont la section est circulaire, carrée ou polygonale.

4. Implant médical modulaire permettant une injection ciblée suivant la revendication **3, caractérisé en ce que** la section du corps (14) est circulaire et dotée de faces planes (141) sur tout ou partie de sa longueur, de façon à pouvoir coopérer avec un outil de serrage.

5. Implant médical modulaire permettant une injection ciblée suivant l'une quelconque des revendications **3** ou **4, caractérisé en ce que** les perforations (31) des modules intermédiaires perforés (3) traversent de la lumière (10) jusqu'à la périphérie du corps (14), permettant la conduction d'un matériau liquide ou pâteux jusqu'aux emplacements ciblés.

6. Implant médical modulaire permettant une injection ciblée suivant la revendication **5**, **caractérisé en ce que** les perforations (31) des modules intermédiaires perforés (3) sont orientées perpendiculairement à la lumière interne (10), ou obliques en étant ascendantes débouchant vers le principe de solidarisation femelle (12) ou descendantes débouchant vers le principe de solidarisation mâle (13).

7. Implant médical modulaire permettant une injection ciblée suivant l'une quelconque des revendications précédentes, **caractérisé en ce que** chaque principe de solidarisation mâle (13) est apte à coopérer avec chaque principe de solidarisation femelle (12).

8. Implant médical modulaire permettant une injection ciblée suivant l'une quelconque des revendications précédentes, **caractérisé en ce que** chaque principe de solidarisation mâle (13) est un filetage mécanique, ou un ou plusieurs ergot(s) périphérique(s), ou tout autre moyen mécanique.

9. Implant médical modulaire permettant une injection ciblée suivant l'une quelconque des revendications précédentes, **caractérisé en ce que** chaque principe de solidarisation femelle (12) est un taraudage mécanique, ou une ou plusieurs gorge(s) périphérique(s), ou tout autre moyen mécanique.

10. Implant médical modulaire permettant une injection ciblée suivant l'une quelconque des revendications précédentes, **caractérisé en ce que** le module proximal (1) et le module distal (2) sont revêtus de filetages à os auto-perforants et auto-taraudants (15, 41).

## Patentansprüche

1. Modulares medizinisches Implantat, das eine gezielte Injektion ermöglicht, gebildet aus einem proximalen Modul (1), einem oder mehreren mittleren Modulen (2), von denen ein oder mehrere perforierte mittlere Module (3) sind, die mit Perforationen (31) ausgestattet sind, und einem distalen Modul (4), **dadurch gekennzeichnet, dass** jedes der Module (1, 2, 3, 4) einen Durchbruch (10) hat und alle Module, die für die Herstellung der gewünschten Länge des modularen medizinischen Implantats notwendig sind, über zusammenwirkende Einheiten miteinander verbunden sind, die ein männliches Verbindungsprinzip (13) und ein weibliches Verbindungsprinzip (12) umfassen, wodurch ermöglicht wird, die Perforationen (31) des oder der perforierten mittleren Module (3) in den erforderlichen Bereichen zu positionieren, um die Injektion eines flüssigen oder pastösen Materials gezielt vorzunehmen.

2. Modulares medizinisches Implantat, das eine gezielte Injektion ermöglicht, nach Anspruch **1, dadurch gekennzeichnet, dass** es Module (1, 2, 3, 4) verschiedener Längen umfasst, um alle Längenmöglichkeiten des endgültigen Implantats und die Auswahl der Positionierung des oder der perforierten Module (3) zu optimieren.

3. Modulares medizinisches Implantat, das eine gezielte Injektion ermöglicht, nach einem der Ansprüche **1** oder **2, dadurch gekennzeichnet, dass** jedes der Module (1, 2, 3, 4), das es bildet, ein Rohr ist, das den Durchbruch (10) definiert und einen Körper (14) hat, dessen Querschnitt kreisförmig, quadratisch oder vieleckig ist.

4. Modulares medizinisches Implantat, das eine gezielte Injektion ermöglicht, nach Anspruch **3, dadurch gekennzeichnet, dass** der Querschnitt des Körpers (14) kreisförmig ist und über seine gesamte oder einen Teil seiner Länge mit ebenen Flächen (141) versehen ist, um mit einem Spannwerkzeug zusammenwirken zu können.

5. Modulares medizinisches Implantat, das eine gezielte Injektion ermöglicht, nach einem der Ansprüche **3** oder **4**, **dadurch gekennzeichnet, dass** die Performationen (31) der perforierten mittleren Module (3) den Durchbruch (10) bis zur Peripherie des Körpers (14) durchqueren, so dass sie die Leitung eines flüssigen oder pastösen Materials bis zu den Zielstellen ermöglichen.

6. Modulares medizinisches Implantat, das eine gezielte Injektion ermöglicht, nach Anspruch **5, dadurch gekennzeichnet, dass** die Perforationen (31) der perforierten mittleren Module (3) senkrecht zum inneren Durchbruch (10) oder schräg ausgerichtet sind, indem sie zum weiblichen Verbindungsprinzip (12) aufsteigend ausmünden oder zum männlichen Verbindungsprinzip (13) absteigend ausmünden.

7. Modulares medizinisches Implantat, das eine gezielte Injektion ermöglicht, nach einem der vorangehenden Ansprüche, **dadurch gekennzeichnet, dass** jedes männliche Verbindungsprinzip (13) imstande ist, mit jedem weiblichen Verbindungsprinzip (12) zusammenzuwirken.

8. Modulares medizinisches Implantat, das eine gezielte Injektion ermöglicht, nach einem der vorangehenden Ansprüche, **dadurch gekennzeichnet, dass** jedes männliche Verbindungsprinzip (13) ein mechanisches Außengewinde oder ein oder mehrere periphere(r) Zapfen oder jedes andere mechanische Mittel ist.

9. Modulares medizinisches Implantat, das eine gezielte Injektion ermöglicht, nach einem der vorangehenden Ansprüche, **dadurch gekennzeichnet, dass** jedes weibliche Verbindungsprinzip (12) ein mechanisches Innengewinde oder eine oder mehrere periphere Ausnehmung(en) oder jedes andere mechanische Mittel ist.

10. Modulares medizinisches Implantat, das eine gezielte Injektion ermöglicht, nach einem der vorangehenden Ansprüche, **dadurch gekennzeichnet, dass** das proximale Modul (1) und das distale Modul (2) mit selbstbohrenden und selbstgewindeformenden Knochengewinden (15, 41) bedeckt sind.

## Claims

1. Modular medical implant for targeted injection consisting of a proximal module (1), one or more intermediate modules (2) of which one or more are perforated intermediate modules (3) provided with perforations (31), and a distal module (4), **characterized in that** each of the modules (1, 2, 3, 4) has a lumen (10) and all the modules required to achieve the desired length of the modular medical implant are connected to each other via cooperating assemblies comprising a male connection principle (13) and a female connection principle (12), thereby enabling the perforations (31) of the perforated intermediate module(s) (3) to be positioned at the levels required to target the injection of a liquid or paste-like material.

2. Modular medical implant for targeted injection according to claim **1, characterised in that** it comprises modules (1, 2, 3, 4) of different lengths, so as to optimise all the possibilities of length of the final implant and the choice of positioning of the perforated module or modules (3).

3. Modular medical implant for targeted injection according to any of claims **1** or **2, characterised in that** each of the modules (1, 2, 3, 4) constituting it is a tube defining the lumen (10) and having a body (14) of circular, square or polygonal cross section.

4. Modular medical implant for targeted injection according to claim **3, characterised in that** the body section (14) is circular and provided with plane faces (141) along all or part of its length, so as to be able to cooperate with a clamping tool.

5. Modular medical implant for targeted injection according to any one of claims **3** or **4, characterised in that** the perforations (31) of the perforated intermediate modules (3) extend from the lumen (10) to the periphery of the body (14), allowing conduction of a liquid or pasty material to the targeted locations.

6. Modular medical implant for targeted injection according to claim **5, characterised in that** the perforations (31) of the perforated intermediate modules (3) are oriented perpendicularly to the inner lumen (10), or obliquely upwards towards the female connection principle (12) or downwards towards the male connection principle (13).

7. Modular medical implant for targeted injection according to any of the preceding claims, **characterised in that** each male attachment principle (13) is adapted to cooperate with each female attachment principle (12).

8. Modular medical implant for targeted injection according to any of the preceding claims, **characterised in that** each male attachment principle (13) is a mechanical thread, or one or more peripheral pins, or any other mechanical means.

9. Modular medical implant for targeted injection according to any of the preceding claims, **characterised in that** each female attachment principle (12) is a mechanical thread, or one or more peripheral grooves, or any other mechanical means.

10. Modular medical implant for targeted injection according to any of the preceding claims, **characterised in that** the proximal module (1) and the distal module (2) are coated with self-piercing and self-tapping bone threads (15, 41).
